(19) Europäisches Patentamt
European Patent Office
Office européen des brevets

(11) Veröffentlichungsnummer: **0 035 728**
**A1**

(12) # EUROPÄISCHE PATENTANMELDUNG

(21) Anmeldenummer: **81101485.1**

(22) Anmeldetag: **02.03.81**

(51) Int. Cl.[3]: **C 07 C 91/12**
C 07 H 15/04, A 61 K 31/13
A 61 K 31/70

(30) Priorität: **10.03.80 AT 1329/80**

(43) Veröffentlichungstag der Anmeldung:
**16.09.81 Patentblatt 81/37**

(84) Benannte Vertragsstaaten:
**BE CH DE FR GB IT LI LU NL SE**

(71) Anmelder: **Henkel Kommanditgesellschaft auf Aktien**
**-Patentabteilung- Postfach 1100 Henkelstrasse 67**
**D-4000 Düsseldorf 1(DE)**

(72) Erfinder: **Fischer, Herbert, Dr.**
**Kamper Weg 139**
**D-4000 Düsseldorf 12(DE)**

(72) Erfinder: **Maak, Norbert, Dr.**
**Liebigstrasse 18**
**D-4040 Neuss(DE)**

(54) **Neue cytostatisch wirksame Desoxy-Amino-Polyol-Verbindungen, diese Verbindungen enthaltende Arzneimittel und ihre Anwendung.**

(57) Di-(2-hydroxy-3-brom-propyl)-substituierte Desoxy-amino-polyole der allgemeinen Formel

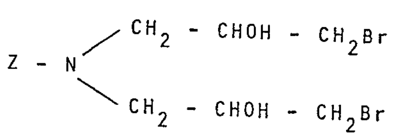

(1)

in der Z der Rest eines Desoxy-amino-polyols mit bis zu 7 C-Atomen ist, wobei auch eine der OH-Gruppen dieses Restes mit der Aldehydgruppe eines monomeren, dimeren oder oligomeren Zuckermoleküls glucosidisch verknüpft sein kann, sowie Arzneimittelzubereitungen mit cytostatischer Wirkung enthaltend disubstituierte Desoxy-Aminopolyole der allgemeinen Formel I und schließlich die Verwendung der Arzneimittelzubereitungen zur Behandlung maligner Neoplasien.

EP 0 035 728 A1

Henkel KGaA
ZR-FE/Patente

6. März 1980

Dr.HF/Gö.

# P a t e n t a n m e l d u n g

D 6095 EP

## Neue cytostatisch wirksame Desoxy-Amino-Polyol-Verbindungen, diese Verbindungen enthaltende Arzneimittel und ihre Anwendung

Es ist seit längerer Zeit bekannt, daß einige alkylierend wirkende Zuckerderivate cytostatisch aktiv sind. So befinden sich Dibromdulcetol und Dibrommannitol im klinischen Test. Diese Produkte erfüllen jedoch durchaus nicht alle Wünsche.

Die vorliegende Erfindung geht von der Feststellung aus, daß in bestimmter Weise substituierte Desoxy-Amino-Polyole eine überraschend starke cytostatische Wirksamkeit entfalten. Diese substituierten Desoxy-Aminopolyole leiten sich dabei insbesondere von Zuckern oder den durch Reduktion aus Zuckern erhaltenen Polyolen ab.

Gegenstand der Erfindung sind dementsprechend in einer ersten Ausführungsform neue Di-(2-hydroxy-3-brom-propyl)-substituierte Desoxy-Aminopolyole der allgemeinen Formel I

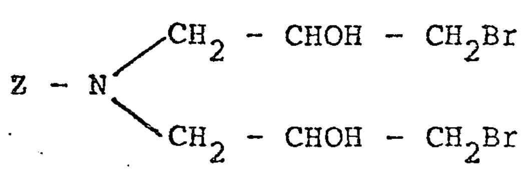

(I)

in der Z der Rest eines Desoxy-Aminopolyols mit bis zu 7 C-Atomen ist. Eine der OH-Gruppen dieses Restes kann mit derAldehydgruppe eines weiteren Zuckermoleküls glycosidisch verknüpft sein. Hierbei erfaßt der Begriff

BAD ORIGINAL

"Zucker" prinzipiell jedes Mono-, Di- oder Oligosaccharid. Für den im folgenden beschriebenen Einsatz als Cytostaticum sind Verbindungen der allgemeinen Formel I ohne zusätzliche glycosidische Verknüpfung mit Zuckermolekülen und solche mit der glycosidischen Verknüpfung mit einem Monosaccharid besonders bevorzugt.

Die neuen Desoxy-Aminopolyole zeichnen sich durch die funktionelle Gruppe II aus

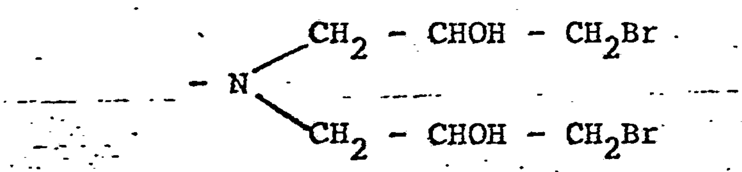

(II)

Diese mit dem 2-Hydroxy-3-brom-propyl-Rest 2-fach substituierte Aminogruppe ist ihrerseits Substituent an dem Rest Z, d. h. an dem Rest eines Desoxy-Polyols. Diese Polyol weist bis zu 7 C-Atome auf und besitzt mindestens 2, vorzugsweise mindestens 3 und in der Regel mindestens 4 Hydroxylgruppen.

Besonders geeignet sind als Träger der funktionellen Gruppe gemäß II Zuckermoleküle bzw. die sich von Zuckern ableitenden Polyole. Ein geeigneter Grundkörper aus der Zuckerreihe ist beispielsweise das Glucoseamin, insbesondere das D-Glucoseamin. Um von ihm zum N-disubstituierten  Derivat zu kommen werden die beiden Wasserstoffatome der $NH_2$-Gruppe durch die Hydroxy-brompropylreste gemäß den Formeln I und II ersetzt.

Besonders bevorzugte Grundkörper für Verbindungen der allgemeinen Formel I sind im Rahmen der Erfindung Desoxy-Aminopolyole der angegebenen Kohlenstoffzahl, die

BAD ORIGINAL

sich von den entsprechenden Zuckern gedanklich durch Reduktion der Carbonylfunktion zur Hydroxylgruppe und Ersatz einer Hydroxylgruppe durch die Aminogruppe ableiten. Besondere Bedeutung besitzen im Rahmen der Erfindung Verbindungen der allgemeinen Formel I, in denen Z der Rest eines 1-Desoxy-1-aminopolyols ist, das von einem Zucker von mit bis zu 7 C-Atomen abgeleitet ist, wobei gewünschtenfalls eine der OH-Gruppen dieses Restes mit der Aldehydgruppe eines monomoren, dimeren oder oligomeren Zuckermoleküls glycosidisch verknüpft sein kann.

Die Erfindung betrifft weiterhin Arzneimittelzubereitungen mit cytostatischer Wirkung, die gekennzeichnet sind durch einen Gehalt an disubstituierten Desoxyaminopolyolen der allgemeinen Formel I. So wurde beispielsweise gefunden, daß N-Di-(2-Hydroxy-3-brompropyl)-glucamin der nachstehenden Formel cytostatisch aktiv ist

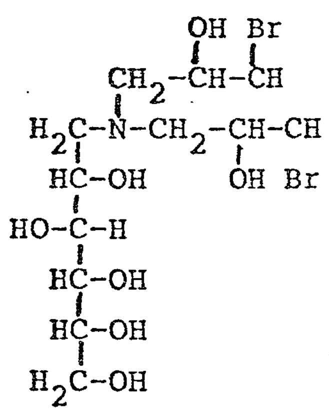

Die T/C-Werte dieser Verbindung bei ihrer Austestung am TumorP 388 (Leukämie) unter Standardprüfbedingungen betragen 166 % (100 mg/kg) bzw. 165 % (50 mg/kg).

BAD ORIGINAL

Andere analoge Derivate, die in den Rahmen der Erfindung fallen und hier besondere Bedeutung besitzen, sind die folgenden:

N,N-Di-(2-hydroxy-3-brom-propyl)-1-desoxy-1-amino-galactose
" " " " " -arabinose
" " " " " -ribose
" " " " " -xylose
" " " " " -maltotriose
" " " " " -lysose
" " " " " -galactose
" " " " " -mannose
" " " " " -glucoheptose
" " " " " -threose
" " " " " -erythrose
" " " " " -maltose
" " " " " -lactose
" " " " " -cellobiose
" " " -2-desoxy-2-amino-fructose

Die erfindungsgemäß eingesetzten Wirkstoffe der allgemeinen Formal I liegen in den erfindungsgemäßen Arzneimittelzubereitungen im allgemeinen zusammen mit üblichen Hilfs- und Trägerstoffen vor. In den Rahmen der Erfindung fällt die Verwendung solcher Arzneimittel zur Behandlung maligner Neoplasien.

Die Herstellung eines Teils der neuen Verbindungen der allgemeinen Formel I erfolgt am einfachsten dadurch, daß man in einem ersten Schritt reduzierende Zucker mit Ammoniak und Wasserstoff zu den Aminozuckern reduziert. Diese Reaktion ist für einige Zucker literaturbekannt, (vergl. US-PSen 2 830 983 und 2 016 962). Die so

BAD ORIGINAL

gewonnenen Aminoalkohole oder andere Aminopolyolverbindungen im Sinne der Verbindung werden mit 2 Mol Epibromhydrin zu den Verbindungen der allgemeinen Formel I umgesetzt, z. B.

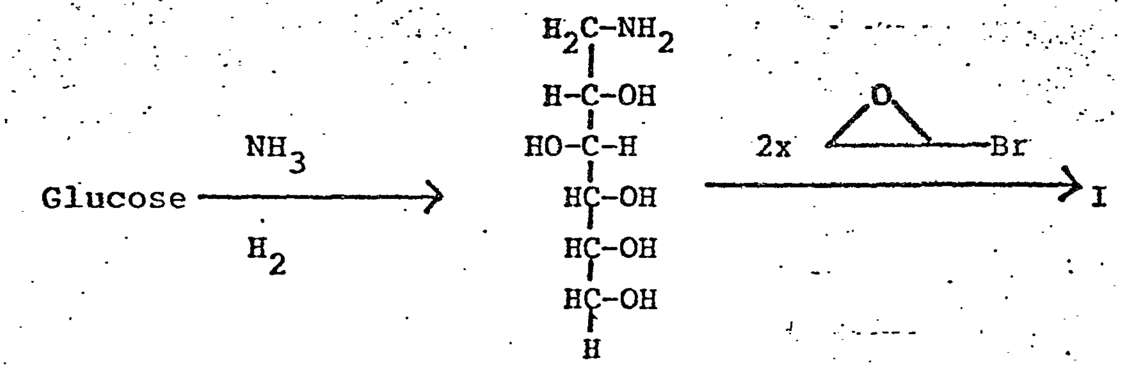


Ersetzt man in den erfindungsgemäßen Verbindungen I die Bromhydrinreste durch entsprechende Chlorhydrinreste so erweisen sich die Verbindungen als unwirksam. Auch das vom N-Methylglucamin abgeleitete N-Methyl-N-(2-hydroxy-3-brom-propyl)-glucamin und die entsprechende 3-Chlorverbindung sind unwirksam.

Es kann angenommen werden, daß die cytostatische Aktivität von Verbindungen der allgemeinen Formel I auf der alkylierenden Wirkung der beiden Bromhydringruppierungen bzw. der sich daraus in situ bildenden Epoxidgruppen beruht. Daher könnten auch anstelle von Verbindungen nach I die entsprechenden Diepoxide eingesetzt werden. Durch Abspaltung von HBr aus I sind sie mit verdünnten Basen zugänglich.

Die erfindungsgemäß eingesetzten Verbindungen der allgemeinen Formel I enthalten in ihrem Rest Z wenigstens 2 vorzugsweise wenigstens 3 und insbesondere wenigstens 4 Kohlenstoffatome auf. Besonders wichtig sind entsprechende Verbindungen mit 5 bis 7 Kohlenstoffatomen im Rest Z. Die erfindungsgemäß eingesetzten Verbindungen

der allgemeinen Formel I treten im allgemeinen in verschiedenen stereoisomeren Formen auf. Grundsätzlich eignen sich alle diese verschiedenen Formen für die Zwecke der Erfindung. Sie können dabei in Mischung oder auch in Form bestimmter isolierter Isomeren eingesetzt werden.

BAD ORIGINAL

Beispiel 1

Herstellung von Di-(2-hydroxy-3-brompropan)-glucamin

9,05 g (0,05 Mol) N-Methylglucamin in 25 ml $H_2O$ und 13,8 g (0,105 Mol) Epibromhydrin werden 5 h bei RT gerührt.
Das Reaktionsprodukt wird in 700 ml Isopropanol von $-20^{\circ}$ C eingerührt und die überstehende Lösung von dem ausgefallenen Brei abdekantiert.
Der Vorgang wird noch einmal wiederholt und die Ausfällung gefriergetrocknet.

| | | |
|---|---|---|
| Auswaage: | 17,1 g eines weißen Sirups | |
| MG: | 380 (ber. 448) | |
| Elemantaranalyse: | C: 34,3 % gef. | 32,2 % ber. |
| | H: 6,0 % gef. | 6,0 % ber. |
| | N: 3,2 % gef. | 3,1 % ber. |
| | Br: 36,8 % gef. | 35,7 % ber. |

Die berechneten Werte sind erhalten aus 1 Mol der Verbindung + 1 Mol Wasser. Das Massenspektrum stützt die Struktur.

Beispiel 2

Die nachfolgenden Versuche wurden durchgeführt nach Testvorschriften des National Cancer Intitute Bethesda, Maryland 200014, veröffentlicht in "Cancer Chemotherapy Reports" Part 3, September 1972, Vol. 3, Nr. 2. Als Wirksubstanz wurde das Di-(2-hydroxy-3-brompropan)-glucamin des Beispiels 1 verwendet. Die Substanz wurde als wäßrige 1-%ige Injektionslösung unmittelbar vor der Applikation frisch hergestellt.

BAD ORIGINAL

Bei Mäusen wurde gemäß Protokoll 1200 (Seite 91.c.) die Tumorart P 388 (Leukämie) i.p. mit $10^6$ Zellen/Maus gesetzt. Die mittlere Überlebensdauer der nicht mit dem erfindungsgemäßen Wirkstoff behandelten Tiere wurde bestimmt.

Einer weiteren Gruppe von Versuchstieren wird der Wirkstoff appliziert. Der Wirkstoff wird dabei in neun Gaben in unterschiedlichen Versuchsreihen einmal zu je 100 mg/kg und in einer weiteren Versuchsreihe zu je 50 mg/kg verabreicht. In allen Fällen wird eine signifikante Verlängerungs der Lebensdauer der behandelten Testtiere gegenüber der Kontrollgruppe der nicht behandelten Tiere erzielt. Die Verlängerungsrate T/C beträgt bei der Verabreichung von 100 mg/kg 166 %, bei der Verabreichung von 50 mg/kg 165 %.

BAD ORIGINAL

# Patentansprüche

## Patentanspruch 1

Di-(2-hydroxy-3-brom-propyl)-substituierte Desoxy-aminopolyole der allgemeinen Formel

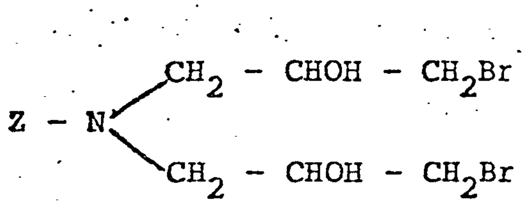

(I)

in der Z der Rest eines Desoxy-amino-polyols mit bis zu 7 C-Atomen ist, wobei auch eine der OH-Gruppen dieses Restes mit der Aldehydgruppe eines monomeren, dimeren oder oligomeren Zuckermoleküls glucosidisch verknüpft sein kann.

## Patentanspruch 2

Verbindungen der allgemeinen Formel I, dadurch gekennzeichnet, daß Z der Rest eines Zuckers oder eines entsprechenden Polyols mit bis zu 7 C-Atomen ist, der gewünschtenfalls mit weiteren Zuckermolekülen glucosidisch verknüpft ist.

## Patentanspruch 3

Verbindungen nach den Ansprüchen 1 und 2, dadurch gekennzeichnet, daß Z der Rest eines sich aus einem Zucker ableitenden 1-Desoxy-1-Aminopolyols ist.

Patentanspruch 4

Arnzeimittelzubereitungen mit cytostatischer Wirkung enthaltend disubstituierte Desoxy-Aminopolyole der allgemeinen Formel I.

Patentanspruch 5

Arzneimittelzubereitungen nach Anspruch 4, dadurch gekennzeichnet, daß sie die Wirkstoffe der allgemeinen Formel I in Abmischung mit üblichen Hilfs- und Trägerstoffen enthalten.

Patentanspruch 6

Verwendung der Arzneimittel gemäß Ansprüchen 4 und 5 zur Behandlung maligner Neoplasien.

BAD ORIGINAL

HENKEL

0035728

KOMMANDITGESELLSCHAFT AUF AKTIEN

Henkel KGaA Postfach 1100 D-4000 Düsseldorf 1

Europäisches Patentamt
Erhardtstraße 27

8000 München 2

1
16 MARS 1981

Henkelstraße 67
Düsseldorf-Holthausen
Telefon (0211) 797-1
Telex Düsseldorf 085817-0
Telegramme henkel düsseldorf

Deutsche Bank AG, Düsseldorf
Konto 2 272 409 (BLZ 300 700 10)
Dresdner Bank AG, Düsseldorf
Konto 2 114 562 (BLZ 300 800 00)
Postscheckkonto Köln 24 14-5 08

81101405.1

Telex (Direktwahl)

| Ihre Nachricht/Zeichen | Unsere Abteilung/Zeichen | Telefon (Direktwahl) | Datum |
|---|---|---|---|
| | ZR-FE/Patente<br>D 6095 EP | 797 3261 | 4. März 1981 |

Neue europäische Patentanmeldung
"Neue cytostatisch wirksame Desoxy-Amino-Polyol-Verbindungen, diese Verbindungen enthaltende Arzneimittel und ihre Anwendung"
Beanspruchte österr. Priorität vom 10. 3.80 - Nr. A 1329/80

Es wird gebeten, bei dem mit Antrag auf Erteilung vom 30. 1.81 übersandten Anmeldungstext folgende offensichtliche Schreibfehler zu berichtigen:

1) S. 1, Z. 3, es muß dort richtig heißen "Dibromdulcitol"
2) S. 4, Z. 10, es muß dort richtig heißen "-lyxose"
3) S. 3, der obere Teil der Formel muß richtig lauten:

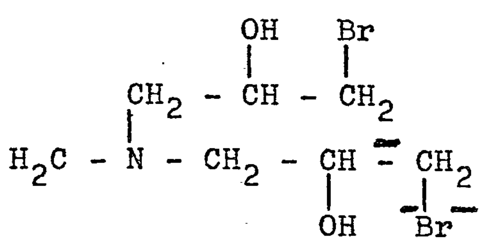

Henkel Kommanditgesellschaft
auf Aktien
ppa. ppa.
Bornemann Dr. Arnoldy
(Allgemeine Vollmacht Nr. 2964)

Der Berichtigung wird stattgegeben
Den Haag den 09. 04. 81
Eingangsstelle
M. Graham
M.W. GRAHAM

Ø Recherchenabt.
01. 04. 81

Aufsichtsratsvorsitzender:
Dr. Konrad Henkel

Zentralgeschäftsführung: Dr. Helmut Sihler (Vorsitzender), Dr. Bruno Werdelmann, Dr. Hans-Otto Wieschermann als persönlich haftende Gesellschafter

Handelsregister:
AG Düsseldorf HRB 4724

0035728

Europäisches Patentamt

# EUROPÄISCHER RECHERCHENBERICHT

Nummer der Anmeldung

EP 81 10 1485

| EINSCHLÄGIGE DOKUMENTE | | | KLASSIFIKATION DER ANMELDUNG (Int Cl ) |
|---|---|---|---|
| Kategorie | Kennzeichnung des Dokuments mit Angabe, soweit erforderlich, der maßgeblichen Teile | betrifft Anspruch | |
| | CHEMICAL ABSTRACTS, Band 78, Nr. 11, 19. März 1973, Seite 28, Nr. 66994u<br>Columbus, Ohio, U.S.A.<br>T. HORVATH et al.: "Synthesis of new sugar derivatives with potential antitumor activity. XVIII. Synthesis of ω,ω'-bis(sulfonyloxalkylamino)-sugar-alcohol, -alkane, and N-substituted aminoalkylthiosulfate derivatives"<br>& MAGY. KEM. LAPJA 1972, 27(7), 361-369<br>* Zusammenfassung *<br>-- | 1,4 | C 07 C 91/12<br>C 07 H 15/04<br>A 61 K 31/13<br>31/70 |
| | | | RECHERCHIERTE SACHGEBIETE (Int. Cl.³) |
| A | ABSTRACT UND CHEMICAL ABSTRACTS, NINTH COLLECTIVE INDEX, volumes 76-85, Chemical Substances G-Hexanol 17279CS:<br>D-Glucitol,<br>-1,6-bis [(2-bromoethyl)amino]-1,6-dideoxy-dihydrobromide [40716-90-3], neoplasm inhibitor<br>-- | 1,4 | C 07 C 91/12<br>C 07 H 15/04<br>A 61 K 31/13<br>31/70 |
| A | DE - A - 1 593 894 (HENKEL)<br>* Anspruch *<br>---- | 1 | |

KATEGORIE DER GENANNTEN DOKUMENTE

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

X Der vorliegende Recherchenbericht wurde für alle Patentansprüche erstellt

| Recherchenort | Abschlußdatum der Recherche | Prüfer |
|---|---|---|
| Den Haag | 04-06-1981 | VERHULST |